# EUROPÄISCHE PATENTSCHRIFT

(11) **EP 4 007 617 B1**
(45) Veröffentlichungstag und Bekanntmachung des Hinweises auf die Patenterteilung: **15.02.2023**
(21) Anmeldenummer: 20749840.3
(22) Anmeldetag: 28.07.2020
(51) Int. Cl.: A61M 1/16

(54) **DIALYSEANLAGE MIT STATIONÄREN WASSERAUFBEREITUNGSEINRICHTUNGEN**
DIALYSIS SYSTEM HAVING FIXED WATER PREPARATION UNITS
SYSTÈME DE DIALYSE COMPORTANT DES UNITÉS DE PRÉPARATION D'EAU FIXES

(30) Priorität: 02.08.2019 DE 102019121003
(43) Veröffentlichungstag der Anmeldung: 08.06.2022
(73) Patentinhaber: B. Braun Avitum AG, 34212 Melsungen (DE)
(72) Erfinder: HILBOLD, Freddy, 79541 Lörrach-Brombach (DE); KRIETEMEYER, Stephan, 79400 Kandern (DE); ODERNHEIMER, Philipp, 34117 Kassel (DE)
(74) Vertreter: Winter, Brandl - Partnerschaft mbB
(86) Internationale Anmeldenummer: PCT/EP2020/071264
(87) Internationale Veröffentlichungsnummer: WO 2021/023579

(56) Entgegenhaltungen:
- DE-A1- 10 256 584
- DE-A1-102008 013 109
- US-A1- 2014 083 917

## Beschreibung

### Technisches Gebiet

Die vorliegende Offenbarung betrifft eine stationäre Dialyseanlage mit wenigstens zwei stationären Wasseraufbereitungseinrichtungen, vorzugsweise der Osmosebauart, an deren jeweiligen Ausgang eine Wasserzuführleitung angeschlossen ist, wenigstens zwei Ringleitungszuläufen, welche eine Vielzahl an selektiv mit stationären und/oder mobilen Dialyseeinheiten fluidkoppelbaren Zweiganschlüssen aufweisen, wenigstens zwei Ringleitungsrückläufe/Ringleitungsrücklaufleitungen/Ablaufleitungen, über welche unverbrauchtes Dialysewasser aus den Dialyseeinheiten zurückführbar ist, und rohrartige Verbindungselemente, welche in einem eingebauten Zustand die Ringleitungen eingangsseitig mit den Wasserzuführleitungen und die Ringleitungen ausgangsseitig mit den Ablaufleitungen fluidisch verbinden.

### Hintergrund der Erfindung

Mit steigender Anzahl der Dialysebehandlungseinheiten werden in stationären Dialyseanlagen auch zunehmend zwei oder mehr Wasseraufbereitungseinrichtungen sowie zwei oder mehr damit verbindbare Ringleitungen installiert. Dabei steht der Gedanke im Vordergrund, dass bei Ausfall einer Wasseraufbereitungseinrichtung oder bei anfälligen Wartungsarbeiten an einer Wasseraufbereitungseinrichtung, die eine oder mehr verbleibende Wasseraufbereitungseinrichtung die Ringleitungen mit Dialysepermeat/Dialyseflüssigkeit versorgen soll. Dafür ist jedoch eine hydraulische Änderung der Einspeisung in die Ringleitungen notwendig bzw. eine hydraulische Änderung der Verbindung zwischen den Wasseraufbereitungseinrichtungen und den Ringleitungen. Bei der Wartung einer Wasseraufbereitungseinrichtung muss diese beispielsweise fluidisch von den Ringleitungen getrennt werden und die verbleibende Wasseraufbereitungseinrichtung versorgt dann die Ringleitungen mit Dialyseflüssigkeit.

Die zu versorgenden Ringleitungen befinden sich dabei zumeist in räumlich voneinander getrennten Behandlungseinheiten (Räume oder Stockwerke). In jeder Behandlungseinheit muss jedoch die Information über den Betriebszustand der angeschlossenen Wasseraufbereitungseinrichtung zur Verfügung stehen, um die Patienten keinem Risiko durch nicht erkannte oder nicht durchgeführte Desinfektionsvorgänge, d.h. verschmutzte Ringleitungen, oder Betriebsstörungen der angeschlossenen medizintechnischen Anlagen, wie z.B. der Wasseraufbereitungseinrichtung oder der Dialyseeinheiten, auszusetzen. Diese Informationsübertragung von den medizintechnischen Anlagen erfolgt üblicherweise auf Fernbedienungen (Remote Control Module), die den Betriebszustand der verbundenen medizintechnischen Anlagen anzeigen können. Fehlende Informationen (beispielsweise über laufende Desinfektionsvorgänge) oder falsch weitergeleitete Information stellen dabei eine erhebliche Patientengefährdung dar.

Bei herkömmlichen stationären Dialyseanlagen und deren Ringleitungsmontagen bzw. Ringleitungsanordnungen sind zwischen den Wasseraufbereitungseinrichtungen und den Ringleitungen manuell schaltbare Hydraulikventile, z.B. 3/2-Wegeventile, verbaut. Durch Schalten dieser Hydraulikventile können somit über eingeleitete Bypässe in die fluidische Verbindung zwischen den Wasseraufbereitungseinrichtungen und den Ringleitungen einzelne Leitungsabschnitte übergangen und somit die Einspeisung in die Ringleitungen verändert werden. Dabei sind jedoch, mathematisch betrachtet, mehr sinnlose Schaltvarianten als sinnvolle Schaltvarianten möglich. Nur die Schaltvarianten bzw. Ventilstellungen, die dem Betreiber auf einer neben einer solchen Ringleitungsmontage ("Ringleitungssplitter") angeordneten Abbildung angezeigt werden, sind erlaubte Schaltvarianten.

Im Allgemeinen müssen bei medizintechnischen Anlagen zudem erkannte Risiken bestmöglich verhindert werden, vorzugsweise durch konstruktive Maßnahmen, die eine Fehlbedienung unmöglich machen (siehe ISO 14971). Optische Maßnahmen zur Darstellung des Risikos, wie z.B. Labeln, reduzieren das Risiko dabei jedoch nicht bis zur Akzeptanzgrenze.

Ein Beispiel einer stationären Dialyseanlage mit lediglich einer stationären Wasseraufbereitungseinrichtung ist in der DE 10 2013 107 673 A1 offenbart. Dabei ist an dem Ausgang der Wasseraufbereitungseinrichtung eine Wasserzuführleitung zur Zufuhr von aufbereitetem Wasser angeschlossen, die wiederum eine Anzahl von Zweiganschlüssen hat, an welche in selektiver Weise Dialysemaschinen fluidgekoppelt sind. Zusätzlich weist die Dialyseanlage eine Ablaufleitung zur Entsorgung verbrauchter Dialyseflüssigkeit auf.

In der EP 2 663 345 B1 hingegen ist eine Dialyseanlage mit einer stationären Herstellungseinheit zum Herstellen von Dialyseflüssigkeit, einem schlauch- oder rohrförmigen Leitungsmittel zum Leiten der Dialyseflüssigkeit und mehreren Dialysebehandlungseinheiten offenbart. Mittels Anschlussmitteln werden die Dialysebehandlungseinheiten an das Leitungsmittel angeschlossen. Über die Schaltung mehrerer in dem Leitungsmittel angeordneter Ventile ist der Zufluss von Dialyseflüssigkeit zu den Dialysebehandlungseinheiten geregelt. In der US 2014 / 0 083 917 A1 und der DE 10 2008 013 109 A1 sind weitere Beispiele für stationäre Dialyseanlagen gezeigt.

Herkömmliche stationäre Dialyseanlagen mit über Hydraulikventilen schaltbaren Ringleitungsanordnungen haben jedoch immer den Nachteil, dass aufgrund nicht angezeigter Desinfektionsvorgänge in den Versorgungsleitungen nach einer Umschaltung zwischen diesen für die Patienten durch verbleibende Desinfektionsflüssigkeit ein erhöhtes Risiko einer Vergiftung besteht. Ferner kann aufgrund von Fehlstellungen der 3/2-Wegeventile fälschlicherweise Desinfektionsflüssigkeit (heiß/chemisch) in die angeschlossenen Ringleitungen eingeleitet werden. Auch mögliche Leckagen der als Kugelhähne ausgeführten Ventile (Erstfehler) können nicht erkannt werden. In den nach Umschaltung nicht durchspülten Leitungsabschnitten können Totzonen entstehen, in welchen sich Dialyseflüssigkeit sammelt und daher leicht Verkeimungen entstehen können.

### Zusammenfassung der Erfindung

Die der vorliegenden Erfindung zugrundeliegende Aufgabe besteht darin, Nachteile des Stands der Technik zu verbessern oder zu beseitigen. Insbesondere soll eine stationäre Dialyseanlage mit einer Mehrzahl an stationären Wasseraufbereitungseinrichtungen, vorzugsweise der Osmosebauart, zur Aufbereitung von Wasser für die Durchführung eines Dialysevorgangs bereitgestellt werden, welche nicht beabsichtigte Betriebszustände ausschließt und somit eine etwaige Gefährdung der Patienten aufgrund möglicher Undichtigkeiten in den Verbindungsleitungen zwischen den Wasseraufbereitungseinrichtungen und den Dialyseeinheiten oder falschem Anschließen der Dialyseeinheiten verhindert. Zudem soll eine sichere Umschaltung der Einspeisung von Dialyseflüssigkeit in wahlweise eine oder mehr Ringleitungen durch eine Person ermöglicht werden und gleichzeitig konstruktiv Totzonen in der Hydraulik (z.B. im Bereich von Kugelhähnen) vermieden werden, um die bakterielle Verkeimung in der Wasserversorgung zu unterbinden. Die erfindungsgemäße Dialyseanlage soll des Weiteren in der Lage sein, Betriebs-und/oder Alarmsignale bei Umschalten der Versorgungsart sicher weiterleiten und anzeigen zu können.

Die der Erfindung zugrundeliegende Aufgabe wird durch eine stationäre Dialyseanlage mit den Merkmalen des Anspruchs 1 gelöst. Vorteilhafte Ausgestaltungen sind Gegenstand der Unteransprüche und werden später genauer beschrieben.

Der Kerngedanke der Erfindung besteht darin, durch unterschiedliche geometrische/hydraulische Ausgestaltung der fluidischen Verbindung zwischen den Wasseraufbereitungseinrichtungen und den Ringleitungen ventillos nur sinnvolle Schaltvarianten/Versorgungsvarianten abzubilden. In anderen Worten ausgedrückt, soll erfindungsgemäß die aus dem Stand der Technik bekannte Hydraulikschalteinrichtung, welche unter Verwendung von manuell oder elektrisch verstellbaren Ventilen zwei oder mehrere separate (stationäre) Wasseraufbereitungseinrichtungen mit zwei oder mehreren separaten (stationären) Ringleitungen in frei wählbaren, unterschiedlichen Kombinationsvarianten/Versorgungsvarianten in Abhängigkeit den Ventilstellungen miteinander fluidverbindet oder trennt, durch eine Anzahl (Mehrzahl) von sogenannten Kopplungsmasken/Kupplungsmasken (Hydraulikschaltkreise) mit jeweils fester, bzw. unveränderbarer (weil ventillos) Kombinationsvariante ersetzt werden, welche in eine (stationäre) Maskenaufnahme (Schaltkreisaufnahme) eingesetzt ist/wird (an welche die Wasseraufbereitungseinrichtungen/-anlagen und Ringleitungen wie ggf. auch Ablaufleitungen ständig angeschlossen sind) und so die Wasseraufbereitungseinrichtungen mit den Ringleitungen und ggf. Ablaufleitungen gemäß dieser einen maskenspezifischen Kombinationsvariante miteinander verbindet oder trennt. Dies bedeutet, dass mehrere Kopplungsmasken bzw. ein Satz von Kopplungsmasken vorgesehen sind bzw. ist, wobei jede Maske jeweils eine (oder maximal zwei) Kopplungsvariante(n) repräsentieren und die Masken in ausgewählter Weise in die Maskenaufnahme (abwechselnd) eingesetzt werden können. In anderen Worten, ist zur fluidischen Verbindung und/oder Trennung der Wasseraufbereitungseinrichtungen mit den Ringleitungen ein Hydraulikschaltkreis aus einer Vielzahl an festen, unveränderbaren und die Versorgungsvarianten repräsentierenden Hydraulikschaltkreise in eine Schaltkreisaufnahme der Dialyseanlage eingesetzt ist, bzw. ist zur fluidischen Verbindung und/oder Trennung der Wasseraufbereitungseinrichtungen mit den Ringleitungen eine Vielzahl an solchen Hydraulikschaltkreisen in die Schaltkreisaufnahme einsetzbar. Dabei ist es bevorzugt, in die Maskenaufnahme wenigstens einen Sensor einzusetzen bzw. zu integrieren, der die jeweils aktuell eingesetzte Maske anhand der darin angeordneten Fluid- oder Verbindungsleitungen oder eines Labels erkennt und ein der jeweiligen, von der aktuellen Maske umgesetzte Kombinationsvariante entsprechendes Signal an die aktuell angeschlossenen (mobilen) Dialyseeinheiten/-maschinen sendet.

Erfindungsgemäß können demzufolge die rohrartigen Verbindungselemente (Fluidleitungen) hierzu auf einer in einem Gehäuse, vorzugsweise einem Schaltschrank (Maskenaufnahme), einbaubaren Montageplatte (Maske) mit als schellenartigen Verbindungsklammern ausgeführten Anschlussstellen für die Wasserzuführleitungen, die Ringleitungen und die Ablaufleitungen angeordnet. Mit anderen Worten, werden die Verbindungselemente auf der Montageplatte vormontiert und diese wird im Anschluss in Form der vorstehend genannten Maske mitsamt den Verbindungselementen in das Gehäuse eingesetzt.

Die Verbindungsklammern sind dabei vorzugsweise in Form einer Schlauchschelle ausgeführt, welche eine über ein Scharnier verbundene Ober- und Unterschale und einen Verriegelungsmechanismus zur Verriegelung der Oberschale relativ zu der Unterschale aufweist. Die Verbindungselemente wiederum können vorzugsweise an ihren Endabschnitten jeweils einen Anschlussflansch aufweisen, welcher beim Einbau der Verbindungselemente in die jeweilige Unterschale eingelegt und nach Umklappen der Oberschale mit dem Verriegelungsmechanismus gesichert werden kann.

Zudem ist es von Vorteil, wenn die Anschlussstellen mit einer, vorzugsweise konstruktiven, Codierung und die Verbindungselemente mit einer der Codierung der Anschlussstellen entsprechenden, vorzugsweise konstruktiven, Codierung ausgestaltet sind. Vorzugsweise kann die Codierung der Anschlussstellen als Rücksprünge und die Codierung der Verbindungselemente als Vorsprünge, welche in dem eingebauten Zustand der Verbindungselemente in die Rücksprünge der Anschlussstellen greifen, ausgeführt sein, so dass fehlerhaftes Einbauen der Montageplatte (Kupplungsmaske) mitsamt den Verbindungselementen durch die konstruktive Ausgestaltung verunmöglicht wird. Eine unerkannte Migration von Desinfektionsmittel in die Ringleitungen wird somit ausgeschlossen.

Des Weiteren ist es vorteilhaft, wenn die erfindungsgemäße Dialyseanlage Sensoren, vorzugsweise Näherungssensoren, zum Detektieren des Hydraulikschaltkreises, insbesondere des eingebauten Zustands der Verbindungselemente bzw. der Kupplungsmaske, sowie eine Steuereinheit aufweist, welche den von den Sensoren detektierten Hydraulikschaltkreis bzw. eingebauten Zustand und somit die Versorgungsvariante erfasst und basierend darauf eine mit wenigstens einer Betriebsmeldeleuchte ausgeführte Anzeigeeinheit steuert. Zusammen mit der konstruktiven Ausgestaltung der Verbindungselemente bzw. der Kupplungsmasken kann durch eine logische Auswertung des Einbauzustands in der Steuereinheit das Patientenrisiko der Vergiftung auf ein Minimum reduziert werden.

Bei der Installation der erfindungsgemäßen Dialyseanlage können die Wasseraufbereitungseinrichtungen und die Dialyseeinheiten vorzugsweise örtlich voneinander getrennt angeordnet sein. Dabei kann es zudem von Vorteil sein, wenn sich die Wasseraufbereitungseinrichtungen und die Dialyseeinheiten in verschiedenen Räumen, insbesondere auf unterschiedlichen Stockwerken, einer Klinik befinden. So kann sichergestellt werden, dass nur qualifiziertes Personal Zugang zu den Wasseraufbereitungseinrichtungen hat.

Gemäß einer bevorzugten Ausgestaltung kann die Dialyseanlage ferner mit einer an den Dialyseeinheiten angeordneten Fernsteuereinheit/Fernbedienung zur Anzeige der Versorgungsvariante bzw. des eingebauten Zustands ausgestattet sein. Dabei kann die Fernsteuereinheit als Bildschirm an jeder Dialyseeinheit ausgeführt sein. Alternativ ist es jedoch auch denkbar, die Fernsteuereinheit als zentrale, insbesondere mobile, Anzeigeeinrichtung, beispielsweise in Form einer mobilen Rechnereinheit, wie einem Tablet-PC, auszuführen. Über die Fernsteuereinheit können somit jederzeit der Einbauzustand und damit die jeweilige Versorgungsvariante der Dialyseanlage sowie etwaige Fehlermeldungen überwacht werden.

In einem bevorzugten Ausführungsbeispiel kann durch die Kupplungsmaske, insbesondere durch die Montageplatte, eine erste Versorgungsvariante realisierbar sein. Zudem kann durch Austauschen der einen Montageplatte mit einer weiteren Montageplatte eine zweite Versorgungsvariante, vorzugsweise auf derselben Maskenaufnahme, realisierbar werden. Dabei ist denkbar, dass durch eine geometrische Veränderung der Ausrichtung der weiteren Montageplatte eine dritte Versorgungsvariante realisiert werden kann. Erfindungsgemäß können die Verbindungselemente in der ersten Versorgungsvariante als sich im Wesentlichen axial erstreckende Rohrleitungen ausgebildet sein und in der zweiten oder dritten Versorgungsvariante als gabelförmiges Rohrstück mit einem Einlass und zwei Auslässen ("Splitversorger") ausgeführt sein, so dass lediglich die für die jeweilige Versorgungsvariante benötigten Rohrleitungen, d.h. in den Wasserzuführleitungen, den Ringleitungen, den Ablaufleitungen, durchströmt werden und somit konstruktionsbedingt keine die Verkeimung begünstigenden Totzonen in den Rohrleitungen entstehen.

### Kurzbeschreibung der Figuren

Nachfolgend wird die vorliegende Erfindung anhand eines bevorzugten Ausführungsbeispiels beschrieben. Dieses ist jedoch nur veranschaulichender Natur und soll den Schutzumfang der vorliegenden Erfindung nicht einschränken.
Fig. 1 ist eine schematische Darstellung einer ersten Versorgungsvariante einer Dialyseanlage gemäß einem bevorzugten Ausführungsbeispiel der vorliegenden Offenbarung;
Fig. 2 ist eine Ansicht einer Montageplatte mit Verbindungselementen gemäß dem bevorzugten Ausführungsbeispiel;
Fig. 3 ist eine Ansicht der Montageplatte mit Verbindungselementen gemäß dem bevorzugten Ausführungsbeispiel im eingebauten Zustand;
Fig. 4 ist eine schematische Darstellung einer Anzeigeeinheit gemäß der vorliegenden Offenbarung;
Fig. 5 ist eine tabellarische Übersicht eines der ersten Versorgungsvariante entsprechenden Anzeigezustands von Betriebsmeldeleuchten der Anzeigeeinheit;
Fig. 6 ist eine schematische Darstellung einer zweiten Versorgungsvariante der Dialyseanlage gemäß dem bevorzugten Ausführungsbeispiel der vorliegenden Offenbarung;
Fig. 7 ist eine tabellarische Übersicht des der zweiten Versorgungsvariante entsprechenden Anzeigezustands der Betriebsmeldeleuchten der Anzeigeeinheit;
Fig. 8 ist eine Ansicht einer Montageplatte mit Verbindungselementen gemäß dem bevorzugten Ausführungsbeispiel für die zweite Versorgungsvariante;
Fig. 9 ist eine schematische Darstellung einer dritten Versorgungsvariante der Dialyseanlage gemäß dem bevorzugten Ausführungsbeispiel der vorliegenden Offenbarung; und
Fig. 10 ist eine tabellarische Übersicht des der dritten Versorgungsvariante entsprechenden Anzeigezustands der Betriebsmeldeleuchten der Anzeigeeinheit.

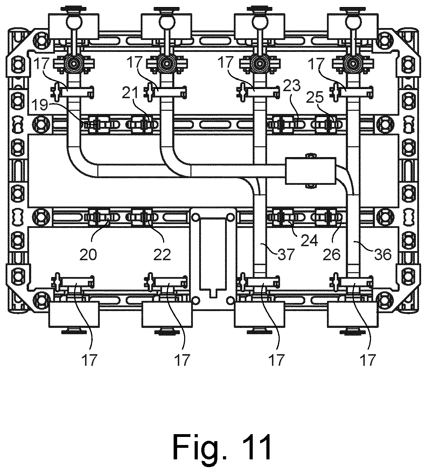

Fig. 11 ist eine Ansicht der Montageplatte mit Verbindungselementen gemäß dem bevorzugten Ausführungsbeispiel im eingebauten Zustand für die dritte Versorgungsvariante;

### Beschreibung des Ausführungsbeispiels

Nachstehend wird ein bevorzugtes Ausführungsbeispiel der vorliegenden Offenbarung auf der Basis der zugehörigen Figuren beschrieben.

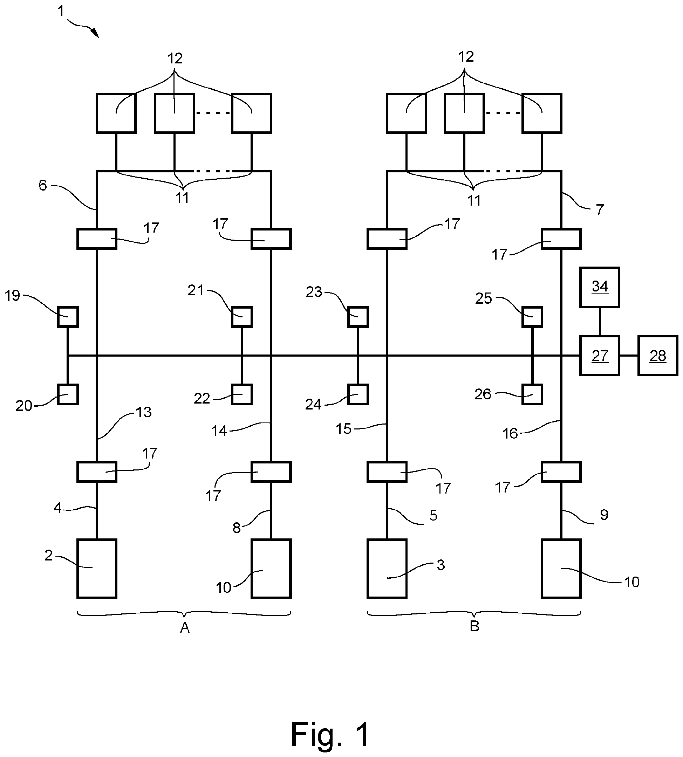

Fig. 1 zeigt eine schematische Darstellung einer ersten Schaltanordnung/Versorgungsvariante einer stationären Dialyseanlage 1 gemäß einem bevorzugten Ausführungsbeispiel der vorliegenden Offenbarung.

In dem bevorzugten Ausführungsbeispiel hat die Dialyseanlage 1 zwei Umkehrosmoseeinheiten 2, 3 zur Wasseraufbereitung, an deren jeweiligem Ausgang eine Wasserzuführleitung 4, 5 angeschlossen ist, über welche das aufbereitete Wasser zu einer als Grundrahmen ausgebildeten Maskenaufnahme geleitet wird. An dieser Maskenaufnahme sind zudem eine erste Ringleitung 6, eine zweite Ringleitung 7 und zwei Ringleitungsrücklaufleitungen 8, 9 zur Rückführung von unverbrauchtem Dialysepermeat in einen Behälter 10 vor der Wasseraufbereitung angeschlossen, so dass das aufbereitete Wasser durch eine Kupplungsmaske innerhalb der Maskenaufnahme auf die erste Ringleitung 6 und die zweite Ringleitung 7 verteilt wird und nach dem Durchströmen dieser über die Kupplungsmaske und die Ringleitungsrückläufe 8, 9 in den Behälter 10 strömen kann. Dabei bilden die Umkehrosmoseeinheit 2, die Wasserzuführleitung 4 und die Ringleitungsrücklaufleitung 8 ein erstes System A aus, und die Umkehrosmoseeinheit 3, die Wasserzuführleitung 5 und die Ringleitungsrücklaufleitung 9 bilden ein zweites System B aus.

In dem bevorzugten Ausführungsbeispiel sind die Umkehrosmoseeinheiten 2, 3 Beispiele für eine erfindungsgemäße "Wasseraufbereitungseinrichtung". Jedoch können selbstverständlich auch andere Wasseraufbereitungseinrichtungen zusätzlich zu oder anstelle der Umkehrosmoseeinheiten 2, 3 verwendet werden.

Entlang der ersten Ringleitung 6 und der zweiten Ringleitung 7 ist jeweils eine Vielzahl an Zweiganschlüssen 11 angeordnet, an welchen, vorzugsweise mobile, Dialyseeinheiten 12 angeschlossen werden können. Die Dialyseeinheiten 12 sind dabei örtlich von den Umkehrosmoseeinheiten 2, 3 entkoppelt und befinden sich beispielsweise in einer Klinik, in welcher die Dialyseanlage 1 eingesetzt wird, auf einem anderen Stockwerk als die Umkehrosmoseeinheiten 2, 3.

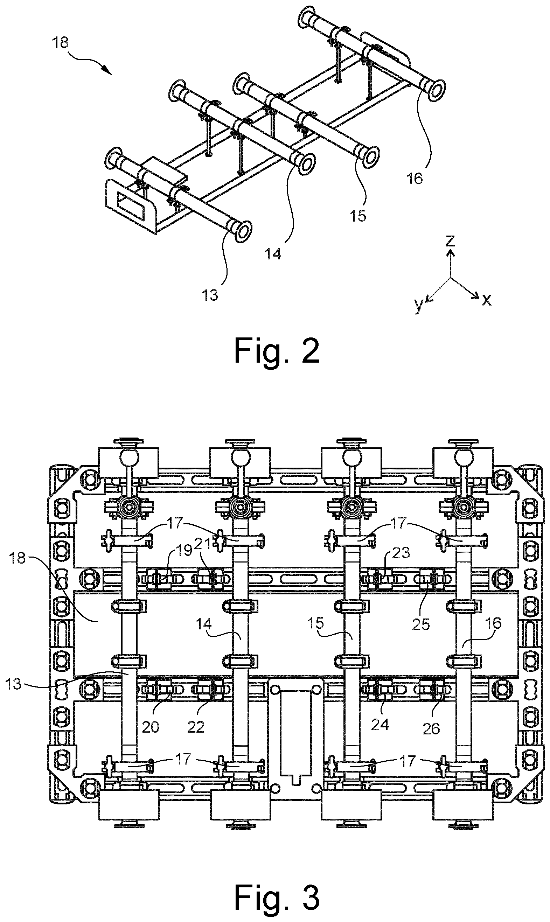

Zur fluidischen Verbindung der Umkehrosmoseeinheiten 2, 3 und der Dialyseeinheiten 12 sowie der Dialyseeinheiten 12 und der Ablaufleitungen 8, 9 werden in die Maskenaufnahme rohrartige, in Fig. 2 gezeigte Verbindungselemente 13, 14, 15, 16 eingebaut, wie in Fig. 3 zu sehen. Hierfür sind ortsfest in der Maskenaufnahme nachstehend beschriebene Verbindungsklammern ("Clampverbinder") 17 zum Anschließen der Verbindungselemente 13, 14, 15, 16 an die Wasserzuführleitungen 4, 5, einen Ein- und Auslass der ersten Ringleitung 6 und der zweiten Ringleitung 7 sowie an die Ringleitungsrücklaufleitungen 8, 9 angeordnet.

Mit anderen Worten, bilden die Verbindungsklammern 17 somit definierte Anschlussstellen zur Herstellung einer fluidischen Verbindung zwischen den Umkehrosmoseeinheiten 2, 3 und den Dialyseeinheiten 12 sowie den Dialyseeinheiten 12 und den Ablaufleitungen 8, 9 beim Einbau der Verbindungselemente 13, 14, 15, 16. In der Dialyseanlage 1 gemäß dem bevorzugten Ausführungsbeispiel sind die Verbindungsklammern 17 so in dem Schaltschrank angeordnet, dass die Verbindungsklammern 17 der Wasserzuführleitung 4 und der Ringleitungsrücklaufleitung 8 den Verbindungsklammern 17 des Ein- bzw. Auslasses der ersten Ringleitung 6 gegenüberliegen und die Verbindungsklammern 17 der Wasserzuführleitung 5 und der Ringleitungsrücklaufleitung 9 den Verbindungsklammern 17 des Ein- bzw. Auslasses der zweiten Ringleitung 7 gegenüberliegen.

Die Verbindungsklammern 17 sind dabei in Form einer Schlauchschelle ausgeführt, welche eine über ein Scharnier verbundene Ober- und Unterschale und einen Verriegelungsmechanismus zur Verriegelung der Oberschale relativ zu der Unterschale aufweist. Die Verbindungselemente 13, 14, 15, 16 wiederum weisen, wie in Fig. 2 zu erkennen, an ihren Endabschnitten jeweils einen Anschlussflansch auf, welcher beim Einbau der Verbindungselemente 13, 14, 15, 16 in die jeweilige Unterschale eingelegt und nach Umklappen der Oberschale mit dem Verriegelungsmechanismus gesichert werden kann.

Des Weiteren weisen die Verbindungsklammern 17 zudem Rücksprünge/Aussparungen auf, in welche an den Verbindungselementen 13, 14, 15, 16 ausgebildete Vorsprünge greifen, sobald diese eingebaut werden. In anderen Worten ausgedrückt, sind die Verbindungsklammern 17 und die Verbindungselemente 13, 14, 15, 16 mittels der Rücksprünge und der Vorsprünge konstruktiv codiert ausgeführt.

Wie in Fig. 2 dargestellt, sind die Verbindungselemente 13, 14, 15, 16 zudem ortsfest auf einer Montageplatte (Kupplungsmaske) 18, welche in der Maskenaufnahme montiert wird, angeordnet. Diese ortsfeste Anordnung auf der Montageplatte 18 sowie die vorstehend erwähnte konstruktive Codierung mittels der Rücksprünge und Vorsprünge lässt nur einen Einbauzustand und damit nur eine Versorgungsvariante zu. Anders ausgedrückt ist aufgrund der Codierung der Verbindungsklammern 17 mechanisch nur ein Einbauzustand der Montageplatte 18 möglich, da sonst die Verbindungsklammern 17 nicht geschlossen werden können.

Um detektieren zu können, ob und welche Verbindungselemente 13, 14, 15, 16 eingebaut wurden, sind in dem Schaltschrank zusätzlich Näherungssensoren 19, 20, 21, 22, 23, 24, 25, 26 angeordnet. Dabei sind die Näherungssensoren 19, 20 im Bereich des Verbindungselements 13 angeordnet, so dass der Näherungssensor 19 einen ringleitungsseitigen Abschnitt des Verbindungselements 13 und der Näherungssensor 20 einen wasserzuführseitigen Abschnitt des Verbindungselements 13 erfassen kann. Dementsprechend sind auch die Näherungssensoren 23, 24 im Bereich des Verbindungselements 15 angeordnet, um einen ringleitungsseitigen Abschnitt des Verbindungselements 15 bzw. einen wasserzuführseitigen Abschnitt des Verbindungselements 15 erfassen zu können. Die Näherungssensoren 21, 22 und 25, 26 sind jeweils im Bereich des Verbindungselements 14 bzw. 16 angeordnet, um ringleitungsseitige Abschnitte bzw. ringleitungsrücklaufleitungsseitige Abschnitte der Verbindungselemente 14 und 16 zu erfassen.

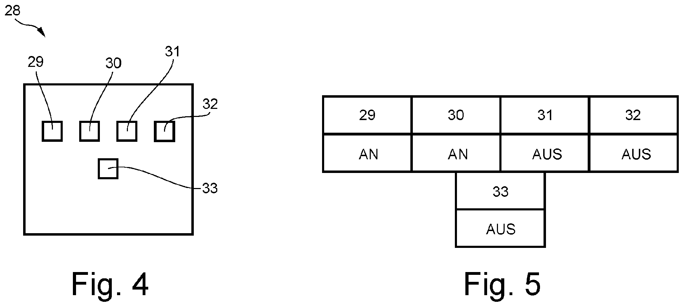

Die Näherungssensoren 19, 20, 21, 22, 23, 24, 25, 26 geben in Abhängigkeit des Detektionsergebnisses, d.h. in Abhängigkeit des Vorhandenseins oder Nichtvorhandenseins des entsprechenden Abschnitts des jeweiligen Verbindungselements 13, 14, 15, 16, Signale an eine Steuereinheit 27 aus. Diese wertet den erfassten Einbauzustand aus und steuert eine in Fig. 4 dargestellte Anzeigeeinheit 28. Diese Anzeigeeinheit 28 ist außerhalb des Schaltschranks, vorzugsweise auf einer Tür des Schaltschranks, angeordnet und weist in dem bevorzugten Ausführungsbeispiel fünf Betriebsmeldeleuchten 29, 30, 31, 32, 33 auf. Die Betriebsmeldeleuchten 29, 30, 31, 32, 33 können dabei jeweils in zwei Betriebszuständen (EIN bzw. "leuchtet"; AUS bzw. "leuchtet nicht") betrieben werden. Die Betriebsmeldeleuchte 29 zeigt dabei an, ob die Dialyseanlage 1 eingeschaltet ist, d.h. mit Strom versorgt wird. Die Betriebsmeldeleuchten 30, 31, 32 zeigen jeweils an, ob die erste Versorgungsvariante, eine zweite Versorgungsvariante oder eine dritte Versorgungsvariante vorliegt, und die Betriebsmeldeleuchte 33 dient zur Fehleranzeige und leuchtet, sobald die Steuereinheit 27 fehlerhafte Signale empfängt.

Neben der Steuerung der Betriebsmeldeleuchten 29, 30, 31, 32, 33 gibt die Steuereinheit 27 zusätzlich Informationen über die Versorgungsvariante an eine an den Dialyseeinheiten 12 ausgebildete Fernsteuereinheit ("Remote control") 34 aus. Diese kann dabei an jeder Dialyseeinheit 12 in Form eines Bildschirms, auf welchem die Informationen der Steuereinheit 27 ausgegeben werden, ausgeführt sein. Alternativ kann die Fernsteuereinheit 34 auch eine zentrale Einheit, welche die Informationen der Steuereinheit 27 ausgibt, sein. So kann die Fernsteuereinheit 34 beispielsweise ein tragbarer Rechner in Form eines Tablet-PCs sein.

In Fig. 5 sind tabellarisch die Betriebszustände der Betriebsmittelleuchten 29, 30, 31, 32, 33 für die erste Versorgungsvariante dargestellt. Dabei leuchten die Betriebsmittelleuchten 29, 30, während die Betriebsmittelleuchten 31, 32, 33 nicht leuchten. Das heißt, die Dialyseanlage 1 ist eingeschaltet und die erste Versorgungsvariante ist gewählt.

Bei der ersten Versorgungsvariante ist, wie in Fig. 1 und Fig. 3 gezeigt, die Umkehrosmoseeinheit 2 bzw. die Wasserzuführleitung 4 über das Verbindungselement 13 mit dem Einlass der ersten Ringleitung 6 fluidisch verbunden. Das Verbindungselement 14 verbindet den Auslass der ersten Ringleitung 6 mit der Ringleitungsrücklaufleitung 8. Die Umkehrosmoseeinheit 3 mit der Wasserzuführleitung 5 ist über das Verbindungselement 15 fluidisch mit dem Einlass der zweiten Ringleitung 7 verbunden, wohingegen das Verbindungselement 16 den Auslass der zweiten Ringleitung 7 mit der Ringleitungsrücklaufleitung 9 verbindet. D.h. die erste Ringleitung 6 wird bei der Versorgungsvariante gemäß dem ersten Ausführungsbeispiel über das erste System A gespeist, während die zweite Ringleitung 7 fluidisch getrennt über das zweite System B versorgt wird.

Wie aus Fig. 1 klar ersichtlich, erfassen alle Näherungssensoren 19, 20, 21, 22, 23, 24, 25, 26 das Vorhandensein der jeweiligen Abschnitte der Verbindungselemente 13, 14, 15, 16 und geben diese Signale an die Steuereinheit 27 weiter.

Mit anderen Worten, speisen in der ersten Versorgungsvariante (Direktversorgung; Standard-Betriebsart) die beiden Umkehrosmoseeinheiten 2, 3 unabhängig voneinander in jeweils eine der beiden hydraulisch getrennte Ringleitungen 6, 7. Dabei speist das System A die erste Ringleitung 6 und das System B die zweite Ringleitung 7. Das erste System A und die erste Ringleitung 6 werden hydraulisch getrennt von dem zweiten System B und der zweiten Ringleitung 7 betrieben. Über die Näherungssensoren 19, 20, 21, 22, 23, 24, 25, 26 wird die Installationsvariante erkannt. An den angeschlossenen Fernsteuereinheiten 34 werden die Betriebsmeldungen (Dialysebetrieb, Desinfektion, Alarm) der Systeme A, B angezeigt.

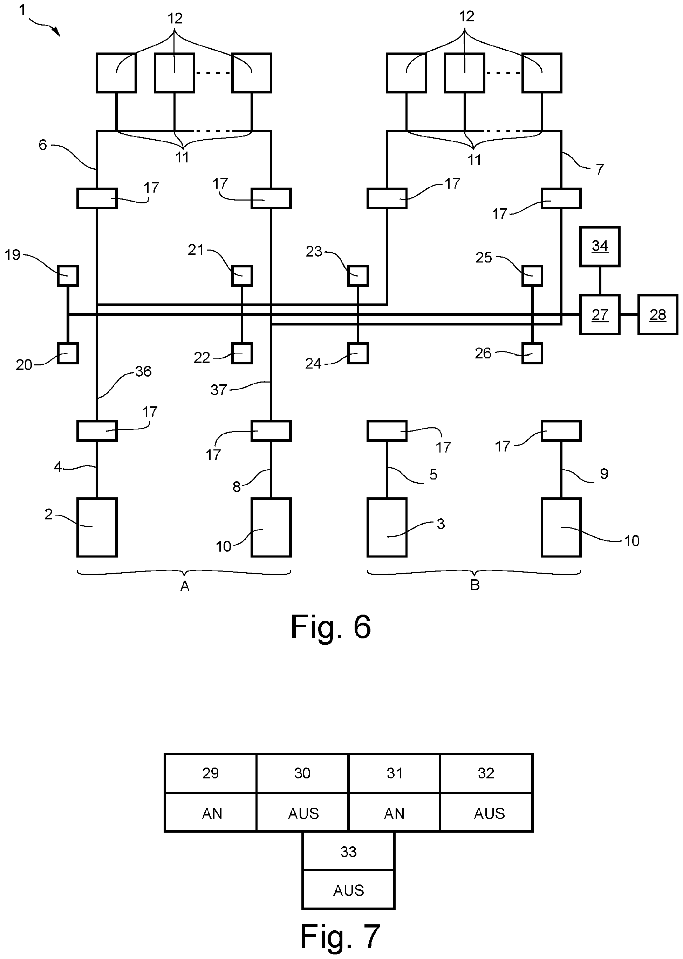

In Fig. 6 ist schematisch die Dialyseanlage 1 gemäß dem bevorzugten Ausführungsbeispiel in der zweiten Versorgungsvariante gezeigt. Im Folgenden wird bei der Beschreibung dieser zweiten Versorgungsvariante lediglich auf die Unterschiede zu der in Fig. 1 gezeigten ersten Versorgungsvariante eingegangen.

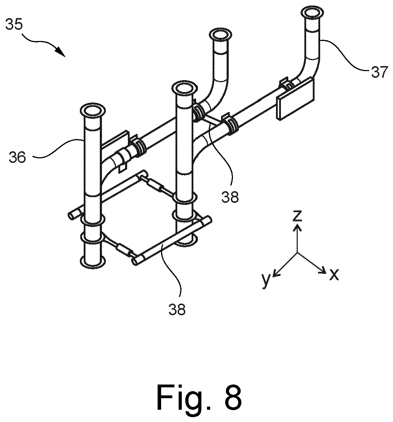

Wenn die Dialyseanlage 1 in der zweiten Versorgungsvariante betrieben wird, wird die Montageplatte 18 mitsamt der Verbindungselemente 13, 14, 15, 16 durch eine Montageplatte 35 mit darauf ortsfest angeordneten Verbindungselementen 36, 37 ausgetauscht. Wie in Fig. 8 dargestellt, sind die Verbindungselemente 36, 37 dabei als gabelartige Rohrstücke mit einem Einlass und zwei Auslässen ("Splitversorger") ausgeführt. Feste Verbindungen 38, beispielweise über eine Schellen-Distanzstangen-Kombination, zwischen den beiden Verbindungselementen 36, 37, welche nur mit einem speziellen Werkzeug gelöst werden können, verhindern, dass nur eines der Verbindungselemente 36, 37 eingebaut werden kann, was zu einer unzulässigen Versorgungsvariante führen würde.

In der zweiten Versorgungsvariante verbindet das Verbindungselement 36 die Wasserzuführleitung 4 mit dem Einlass der ersten Ringleitung 6 und dem Einlass der zweiten Ringleitung 7. Das Verbindungselement 37 hingegen verbindet die Ringleitungsrücklaufleitung 8 mit dem Auslass der ersten Ringleitung 6 und dem Auslass der zweiten Ringleitung 7. Die nicht verbundenen Verbindungsklammern 17 des zweiten Systems B, d.h. die Verbindungsklammer 17 an der Wasserzuführleitung 5 und der Ringleitungsrücklaufleitung 9, werden durch Blindstücke geschlossen.

In der zweiten Versorgungsvariante detektieren folglich die Näherungssensoren 19, 20, 21, 22, 23 und 25 ein vorhandenes Verbindungselement 36 bzw. 37, wohingegen die beiden Näherungssensoren 24, 26 das Nichtvorhandensein eines Verbindungselements erfassen. Diese Detektionsergebnisse senden die Näherungssensoren 19, 20, 21, 22, 23, 24, 25, 26, analog zur ersten Versorgungsvariante, an die Steuereinheit 27, welche basierend darauf wiederum die Anzeigeeinheit 28 mitsamt der Betriebsmeldeleuchten 29, 30, 31, 32, 33 steuert. Die Betriebszustände der Betriebsmeldeleuchten 29, 30, 31, 32, 33 in der zweiten Versorgungsvariante sind in Fig. 7 tabellarisch dargestellt. Dabei leuchten die Betriebsmittelleuchten 29, 31, während die Betriebsmittelleuchten 30, 32, 33 nicht leuchten. Das heißt, die Dialyseanlage 1 ist eingeschaltet und die zweite Versorgungsvariante ist gewählt.

Mit anderen Worten, wird durch Austauschen der Montageplatte 18 und Einsetzen der Montageplatte 35 das erste System A zur Einspeisung des Dialysepermeats in die erste Ringleitung 6 und die zweite Ringleitung 7 verwendet. Die offenen Verbindungsklammern 17 des zweiten Systems B werden durch Blindstücke verschlossen. Die Näherungssensoren 19, 20, 21, 22, 23, 24, 25, 26 erkennen die zweite Versorgungsvariante als zulässige, erlaubte Versorgungsvariante. Die theoretisch denkbare Versorgungsvariante einer einzelnen Ringleitung, d.h. der ersten Ringleitung 6 oder der zweiten Ringleitung 7, durch das erste System A und das zweite System B (entspricht in Fig. 8 einer Drehung der Montageplatte 35 um 180° um die x-Achse) ist durch die konstruktive Codierung der Verbindungsklammern 17 über Rück-und Vorsprünge mechanisch verhindert. Wie vorstehend erwähnt, ist auch eine Einzelverwendung der beiden Verbindungselemente 36, 37 durch die feste Verbindung 38 zwischen den Verbindungselementen 36, 37 ausgeschlossen.

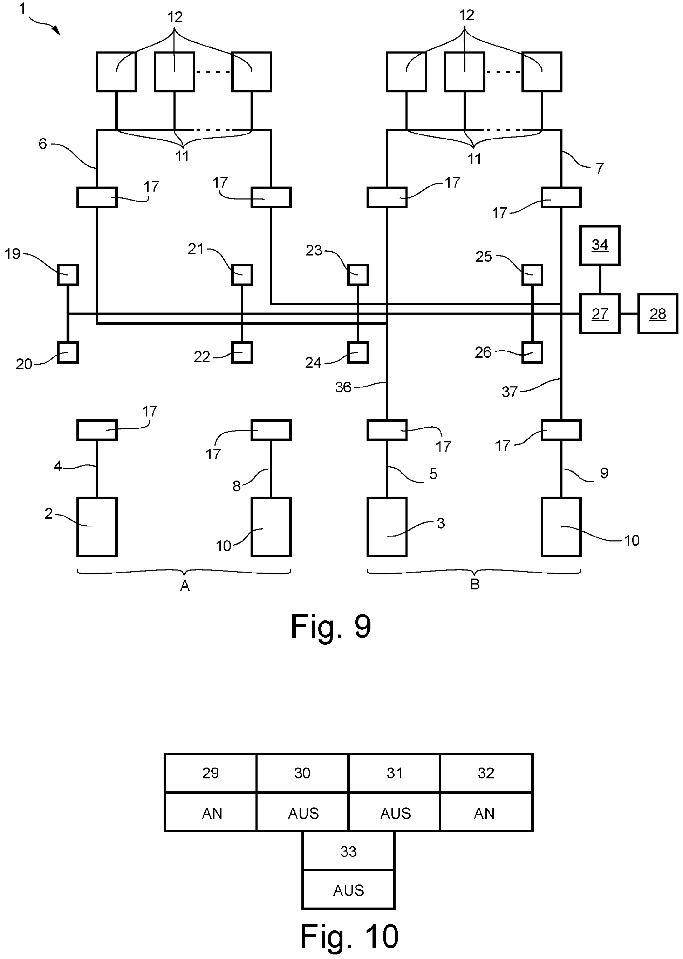

In Fig. 9 ist schematisch die Dialyseanlage 1 gemäß dem bevorzugten Ausführungsbeispiel in der dritten Versorgungsvariante gezeigt. Im Folgenden wird bei der Beschreibung dieser dritten Versorgungsvariante lediglich auf die Unterschiede zu der in Fig. 6 gezeigten zweiten Versorgungsvariante eingegangen.

Wenn die Dialyseanlage 1 in der dritten Versorgungsvariante betrieben wird, wird die Montageplatte (Kupplungsmaske) 35 mit darauf ortsfest angeordneten Verbindungselementen 36, 37 im Vergleich zu der zweiten Versorgungsvariante um 180° entlang ihrer Hochachse (entspricht der z-Achse in Fig. 8) gewendet und daraufhin, wie in Fig. 10 gezeigt, eingesetzt.

Somit verbindet in der dritten Versorgungsvariante das Verbindungselement 36 die Wasserzuführleitung 5 mit dem Einlass der ersten Ringleitung 6 und dem Einlass der zweiten Ringleitung 7. Das Verbindungselement 37 hingegen verbindet die Ringleitungsrücklaufleitung 9 mit dem Auslass der ersten Ringleitung 6 und dem Auslass der zweiten Ringleitung 7. Die nicht verbundenen Verbindungsklammern 17 des zweiten Systems A, d.h. die Verbindungsklammer 17 an der Wasserzuführleitung 4 und der Ringleitungsrücklaufleitung 8, werden durch Blindstücke geschlossen.

In der zweiten Versorgungssituation detektieren somit die Näherungssensoren 19, 21, 23, 24, 25 und 26 ein vorhandenes Verbindungselement 36 bzw. 37, wohingegen die beiden Näherungssensoren 20, 22 das Nichtvorhandensein eines Verbindungselements erfassen. Diese Detektionsergebnisse senden die Näherungssensoren 19, 20, 21, 22, 23, 24, 25, 26, analog zur ersten bzw. zweiten Versorgungsvariante, an die Steuereinheit 27, welche basierend darauf wiederum die Anzeigeeinheit 28 mitsamt der Betriebsmeldeleuchten 29, 30, 31, 32, 33 steuert. Die Betriebszustände der Betriebsmeldeleuchten 29, 30, 31, 32, 33 in der zweiten Versorgungsvariante sind in Fig. 9 tabellarisch dargestellt. Dabei leuchten die Betriebsmittelleuchten 29, 32, während die Betriebsmittelleuchten 30, 31, 32 nicht leuchten. Das heißt, die Dialyseanlage 1 ist eingeschaltet und die dritte Versorgungsvariante ist gewählt.

Mit anderen Worten, kann durch Wenden der Montageplatte 35 das zweite System B zur Einspeisung des Dialysepermeats in die erste Ringleitung 6 und die zweiten Ringleitung 7 verwendet werden. Die offenen Verbindungsklammern 17 des ersten Systems A werden durch Blindstücke verschlossen.

Unzulässige Versorgungsvarianten, z.B. Einzelverwendung eines Verbindungselements 36, 37, werden durch die konstruktive Codierung der Verbindungsklammern 17 und die feste Verbindung 38 zwischen den Verbindungselementen 36, 37 verhindert sowie durch das Leuchten der Betriebsmeldeleuchte 33 angezeigt. Die Umkehrosmoseeinheiten 2, 3 der Dialyseanlage 1 werden in diesem Fall in einen Fehlerbetrieb geschaltet.

### Bezugszeichenliste

- 1: Dialyseanlage
- 2, 3: Umkehrosmoseeinheit
- 4, 5: Wasserzuführleitung
- 6: erste Ringleitung
- 7: zweite Ringleitung
- 8, 9: Ringleitungsrücklaufleitung
- 10: Ablauf
- 11: Zweiganschluss
- 12: Dialyseeinheit
- 13, 14, 15, 16, 36, 37: Verbindungselement
- 17: Verbindungsklammer
- 18, 35: Montageplatte (Kupplungsmaske)
- 19, 20, 21, 22, 23, 24, 25, 26: Näherungssensor
- 27: Steuereinheit
- 28: Anzeigeeinheit
- 29, 30, 31, 32, 33: Betriebsmeldeleuchte
- 34: Fernsteuereinheit
- 38: feste Verbindung
- A: erstes System
- B: zweites System

## Patentansprüche

1. Stationäre Dialyseanlage (1) mit
wenigstens zwei separaten Wasseraufbereitungseinrichtungen (2, 3), welche gemäß auswählbarer Versorgungsvarianten mit wenigstens zwei separaten Ringleitungen (6, 7) fluidisch verbindbar und/oder trennbar sind,
**dadurch gekennzeichnet, dass**
zur fluidischen Verbindung und/oder Trennung der Wasseraufbereitungseinrichtungen (2, 3) mit den Ringleitungen (6, 7) ein Hydraulikschaltkreis aus einer Vielzahl an festen, unveränderbaren und die Versorgungsvarianten repräsentierenden Hydraulikschaltkreise in eine Schaltkreisaufnahme der Dialyseanlage (1) eingesetzt ist.

2. Dialyseanlage (1) nach Anspruch 1 ferner mit
wenigstens zwei separaten Ringleitungsrücklaufleitungen (8, 9), welche über die Hydraulikschaltkreise mit den Ringleitungen (6, 7) verbindbar sind.

3. Dialyseanlage (1) nach Anspruch 1 oder 2, **dadurch gekennzeichnet, dass** die Hydraulikschaltkreise als fest auf einer Montageplatte (18; 35) angeordnete rohrartige Verbindungselemente (13, 14, 15, 16; 36, 37) ausgestaltet sind.

4. Dialyseanlage nach Anspruch 3, **dadurch gekennzeichnet, dass** die Montageplatte (18; 35) mitsamt den Verbindungselementen (13, 14, 15, 16; 36, 37) in einem als Schaltkreisaufnahme ausgebildeten Grundrahmen mit als Verbindungsklammern (17) ausgeführten Anschlussstellen für die Ringleitungen (6, 7), die Ablaufleitungen (8, 9) und für an den Wasseraufbereitungseinrichtungen (2, 3) angeschlossenen Wasserzuführleitungen (4, 5) eingesetzt ist.

5. Dialyseanlage (1) nach Anspruch 4, **dadurch gekennzeichnet, dass** die Anschlussstellen (17) mit einer Codierung und die Verbindungselemente (13, 14, 15, 16; 36, 37) mit einer der Codierung der Anschlussstellen (17) entsprechenden Codierung ausgestaltet sind.

6. Dialyseanlage (1) nach Anspruch 5, **dadurch gekennzeichnet, dass** die Codierung der Anschlussstellen (17) als Rücksprünge und die Codierung der Verbindungselemente (13, 14, 15, 16; 36, 37) als Vorsprünge, welche in dem eingebauten Zustand der Verbindungselemente (13, 14, 15, 16; 36, 37) in die Rücksprünge der Anschlussstellen (17) greifen, ausgeführt ist.

7. Dialyseanlage (1) nach einem der Ansprüche 1 bis 6, ferner mit Sensoren (19, 20, 21, 22, 23, 24, 25, 26) zum Detektieren des Hydraulikschaltkreises und der Versorgungsvariante.

8. Dialyseanlage (1) nach Anspruch 7, ferner mit einer Steuereinheit (27), welche den von den Sensoren (19, 20, 21, 22, 23, 24, 25, 26) detektierten, eingebauten Zustand erfasst und basierend darauf eine mit wenigstens einer Betriebsmeldeleuchte (29, 30, 31, 32, 33) ausgestattete Anzeigeeinheit (28) steuert.

9. Dialyseanlage (1) nach einem der Ansprüche 1 bis 8, **dadurch gekennzeichnet, dass** die Wasseraufbereitungseinrichtungen (2, 3) und an den Ringleitungen (6, 7) angeschlossene Dialyseeinheiten (12) örtlich voneinander getrennt angeordnet sind.

10. Dialyseanlage (1) nach einem der Ansprüche 1 bis 9, ferner mit einer an den Dialyseeinheiten (12) angeordneten oder zentralen Fernsteuereinheit (34) zur Anzeige der Versorgungsvariante.

## Claims

1. A stationary dialysis system (1) comprising
at least two separate water preparation units (2, 3), which can be fluidically connected and/or separated to/from at least two separate ring lines (6, 7) according to selectable supply variants,
**characterized in that**
a hydraulic circuit of a plurality of fixed, unchangeable hydraulic circuits representing the supply variants is inserted in a circuit receiving means of the dialysis system (1) for fluidically connecting and/or separating of the water preparation units (2, 3) to/from the ring lines (6, 7).

2. The dialysis system (1) according to claim 1, further comprising
at least two separate ring line return lines (8, 9), which can be connected to the ring lines (6, 7) via the hydraulic circuits.

3. The dialysis system (1) according to claim 1 or 2, **characterized in that** the hydraulic circuits are designed as tubular connecting elements (13, 14, 15, 16; 36, 37) arranged fixedly on a mounting plate (18; 35).

4. The dialysis system (1) according to claim 3, **characterized in that** the mounting plate (18; 35) together with the connecting elements (13, 14, 15, 16; 36, 37) is inserted in a main frame formed as a circuit receiving means with connection points designed as connection clips (17) for the ring lines (6, 7), the drain lines (8, 9) and for water supply lines (4, 5) connected to the water preparation units (2, 3).

5. The dialysis system (1) according to claim 4, **characterized in that** the connection points (17) are designed with a coding and the connecting elements (13, 14, 15, 16; 36, 37) are designed with a coding corresponding to the coding of the connection points (17).

6. The dialysis system (1) according to claim 5, **characterized in that** the coding of the connection points (17) is designed as recesses and the coding of the connecting elements (13, 14, 15, 16; 36, 37) is designed as projections which, in the installed state of the connecting elements (13, 14, 15, 16; 36, 37), engage in the recesses of the connection points (17).

7. The dialysis system (1) according to any one of claims 1 to 6, further comprising sensors (19, 20, 21, 22, 23, 24, 25, 26)for detecting the hydraulic circuit and the supply variant.

8. The dialysis system (1) according to claim 7, further comprising a control unit (27) which detects the installed state detected by the sensors (19, 20, 21, 22, 23, 24, 25, 26) and, based thereon, controls a display unit (28) equipped with at least one operating indicator light (29, 30, 31, 32, 33).

9. The dialysis system (1) according to any one of claims 1 to 8, **characterized in that** the water preparation units (2, 3) and dialysis units (12) connected to the ring lines (6, 7) are arranged in such a way that they are locally separated from one another.

10. The dialysis system (1) according to any one of claims 1 to 9, further comprising a remote control unit (34) which is central or arranged on the dialysis units (12) for displaying the supply variant.

## Revendications

1. Installation de dialyse fixe (1) comprenant
au moins deux dispositifs de traitement des eaux usées séparées (2, 3), qui peuvent être reliés et/ou séparés de manière fluidique à au moins deux conduites annulaires séparées (6, 7) selon des variantes d'approvisionnement sélectionnables,
**caractérisée en ce que**
pour relier et/ou séparer de manière fluidique les dispositifs de traitement des eaux usées (2, 3) avec les conduites annulaires (6, 7) un circuit hydraulique issu d'une pluralité de circuits hydrauliques fixes, non modifiables et représentant les variantes d'approvisionnement est inséré dans un logement de circuit de l'installation de dialyse (1).

2. Installation de dialyse (1) selon la revendication 1 comprenant en outre
au moins deux conduites de retour pour conduite annulaire séparées (8, 9), qui peuvent être reliées aux conduites annulaires (6, 7) par l'intermédiaire des circuits hydrauliques.

3. Installation de dialyse (1) selon la revendication 1 ou 2, **caractérisée en ce que** les circuits hydrauliques sont configurés en tant qu'éléments de liaison tubulaires (13, 14, 15, 16 ; 36, 37) disposés de manière fixe sur une plaque de montage (18 ; 35).

4. Installation de dialyse selon la revendication 3, **caractérisée en ce que** la plaque de montage (18 ; 35) est insérée avec les éléments de liaison (13, 14, 15, 16 ; 36, 37) dans un cadre de base réalisé en tant que logement de circuit avec des points de raccordement réalisés en tant que pinces de liaison (17) pour les conduites annulaires (6, 7), les conduites d'évacuation (8, 9) et pour des conduites d'alimentation en eau (4, 5) raccordées aux dispositifs de traitement des eaux usées (2, 3).

5. Installation de dialyse (1) selon la revendication 4, **caractérisée en ce que** les points de raccordement (17) sont conçus avec un codage et les éléments de liaison (13, 14, 15, 16 ; 36, 37) sont configurés avec un codage correspondant au codage des points de raccordement (17).

6. Installation de dialyse (1) selon la revendication 5, **caractérisée en ce que** le codage des points de raccordement (17) est réalisé en tant que parties en retrait et le codage des éléments de liaison (13, 14, 15, 16 ; 36, 37) est réalisé en tant que saillies qui s'engrènent dans les parties en retrait des points de raccordement (17) à l'état monté des éléments de liaison (13, 14, 15, 16 ; 36, 37).

7. Installation de dialyse (1) selon l'une quelconque des revendications 1 à 6, comprenant en outre des capteurs (19, 20, 21, 22, 23, 24, 25, 26) pour détecter le circuit hydraulique et la variante d'approvisionnement.

8. Installation de dialyse (1) selon la revendication 7, comprenant en outre une unité de commande (27) qui acquiert l'état monté détecté par les capteurs (19, 20, 21, 22, 23, 24, 25, 26) et sur cette base commande une unité d'affichage (28) équipée d'au moins un témoin lumineux de fonctionnement (29, 30, 31, 32, 33).

9. Installation de dialyse (1) selon l'une quelconque des revendications 1 à 8, **caractérisée en ce que** les dispositifs de traitement des eaux usées (2, 3) et les unités de dialyse (12) raccordées aux conduites annulaires (6, 7) sont disposés localement et séparés les uns des autres.

10. Installation de dialyse (1) selon l'une quelconque des revendications 1 à 9, comprenant en outre une unité de commande à distance (34) disposée au niveau des unités de dialyse (12) ou centrale pour afficher les variantes d'approvisionnement.
